# EUROPEAN PATENT SPECIFICATION

(11) **EP 3 290 019 B1**
(45) Date of publication and mention of the grant of the patent: **06.03.2019**
(21) Application number: 16186818.7
(22) Date of filing: 01.09.2016
(51) Int. Cl.: A61K 8/365, A61Q 5/04, A61Q 5/06

(54) **HAIR TREATMENT METHOD**
HAARBEHANDLUNGSVERFAHREN
PROCÉDÉ DE TRAITEMENT CAPILLAIRE

(43) Date of publication of application: 07.03.2018
(73) Proprietor: KAO CORPORATION, Tokyo 103-8210 (JP)
(72) Inventor: Nagasaki, Ayaka, Tokyo, 131-8501 (JP); Herzog-Renker, Beate, 64297 Darmstadt (DE)
(74) Representative: Hoffmann Eitle

(56) References cited:
- US-A1- 2013 306 095

## Description

### Field of the Invention

The present invention relates to a method for semipermanently straightening hair without generation of a distinct odor.

### Background of the Invention

Use of a high temperature heating device such as a hair iron is known as a method for temporarily straightening curly hair or frizzy hair. High temperature from the high temperature heating device breaks and restructures hydrogen bonds of hair keratin to thereby achieve temporary hair straightening. These hydrogen bonds are broken by moisture action. Thus, the original hair shape is recovered by moisture in air over time, or the hair-straightening effect is lost by hair washing.

An exemplary method for keeping a changed hair shape permanently or semipermanently is a method comprising allowing a reducing agent having, for example, a sulfide group, a thiol group to act on disulfide bonds to be cleaved, changing the hair shape into a desired shape, and subsequently applying an oxidizing agent such as hydrogen peroxide to restructure the disulfide bonds to thereby fix the hair shape. An alternative method is a method comprising allowing an alkali agent such as sodium hydroxide or guanidine hydroxide to act on disulfide bonds at a pH of about 12 or more to thereby cleave the bonds to generate α,β-unsaturated dehydroalanine moieties and cysteine moieties, and forming thioether bonds to thereby produce lanthionine. These methods can achieve permanent or semipermanent hair straightening, and the hair straightening effect will be lost only by growing of the hair. However, these treatments are known to damage hair.

It has been found recently that combination of a carboxylic acid compound having a carbonyl group adjacent to a carboxyl group (for example, glyoxylic acid) with a mechanical hair straightening means using heating (for example, a hair iron) provides a semipermanent hair straightening effect (for example, Patent Literature 1). This treatment method combining glyoxylic acid with a mechanical hair straightening means using heating (for example, a hair iron) may be followed by a hair dye treatment or bleaching treatment (Patent Literatures 2 and 3). Such hair treatments employing a specific carboxylic acid compound are capable of hair straightening while preventing hair damages and of maintaining the straightened hair appearance even after hair washing is repeated for several times.

### Citation Listing

### [Patent Documents]

[Patent Literature 1] JP-A-2013-520468
[Patent Literature 2] JP-A-2015-535268
[Patent Literature 3] JP-A-2015-535269
   US 2013/306095 describes a method for enhancing the removal of natural curl from hair having a visibly wavy to tight curly configuration, the method comprising, inter alia, the treatment with an alkaline composition to partially straighten the hair, followed by the treatment with an acidic composition comprising glyoxylic acid, a glyoxylic amide or a mixture thereof and the subsequent application of a heated iron.

### Summary of the Invention

The present invention provides a hair treatment method comprising steps 1 to 5 to be performed in the following order:
1. a step of applying a first composition to hair, wherein the first composition is acidic and comprises one or more selected from the group consisting of glyoxylic acid, glyoxylic acid hydrates, glyoxylates, and glyoxyl amides;
2. a step of leaving the hair having the first composition applied thereto to stand;
3. a step of heating the hair with a hair iron having a temperature of 130 to 250°C;
4. a step of applying a second composition to the hair, wherein the second composition comprises substantially no oxidizing agent, i.e., the content of oxidizing agent being less than 0.5% by mass, and has a pH of 9.0 or more;
5. a step of leaving the hair having the second composition applied thereto to stand.

The present invention also provides the use of a hair treatment kit comprising a first composition which is acidic and comprises one or more member selected from the group consisting of glyoxylic acid, glyoxylic acid hydrates, glyoxylates, and glyoxyl amides, and a second composition which comprises substantially no oxidizing agent, i.e., the content of oxidizing agent being less than 0.5% by mass, and has a pH of 9.0 or more, for straightening hair using the procedure according to the above-described method.

### Detailed Description of the Invention

It has been found that hair actually treated by the hair treatment method described in Patent Literature 1 may generate a distinct odor in some cases. This odor is not perceived when the hair is dry, and perceived only when the hair is in a wet condition. Furthermore, this odor is generated not only immediately after the treatment but also for as long as several weeks when the hair becomes wet. Thus, this distinct odor may be perceived whenever the hair becomes wet by, for example, hair washing after the treatment, perspiration. The hair treatment method of Patent Literature 2 or 3 could not sufficiently prevent emission of the distinct odor either. Furthermore, the method of Patent Literature 2 or 3 has been an unsuitable treatment for consumers who desire hair straightening but do not desire change in the color of the hair because the color of the hair is changed after the treatment.

Accordingly, the present invention relates to a hair treatment method comprising a hair straightening treatment employing a glyoxylic acid compound and a high temperature heating device, wherein a distinct odor is not generated from hair not only immediately after the treatment but also for a long period and wherein furthermore, damage to the hair is unlikely to occur.

The present inventor has found, as a result of extensive studies, that after a hair straightening treatment employing a glyoxylic acid compound and a high temperature heating device such as a hair iron, application of an alkaline composition which comprises substantially no oxidizing agent to hair dramatically reduces a distinct odor generated when the hair becomes wet after the treatment, having completed the invention.

### First Composition (Hair Straightening Composition)

The first composition is used for the hair straightening treatment and comprises one or more of compounds selected from the group consisting of glyoxylic acid, glyoxylic acid hydrates, glyoxylates, and glyoxyl amides (hereinafter, may be referred to as glyoxylic acid compound(s)) as an active component. Examples of the glyoxylic acid hydrate include glyoxylic acid monohydrate. Examples of the glyoxylate include alkali metal glyoxylates and alkaline earth metal glyoxylates. Examples of the alkali metal salt include lithium salt, sodium salt, and potassium salts, and examples of the alkaline earth metal salt include magnesium salts and calcium salts. Examples of the glyoxyl amide include N-glyoxyloyl carbocysteine and N-glyoxyloyl keratin amino acids. Of these, glyoxylic acid is preferred.

The content of the glyoxylic acid compound in the first composition is, based on the total amount of the first composition, preferably 0.1% by mass or more, more preferably 0.5% by mass or more, still more preferably 1% by mass or more, still more preferably 2.5% by mass or more, in view of the sufficient hair-straightening effect, and also preferably 40% by mass or less, more preferably 30% by mass or less, still more preferably 25% by mass or less, still more preferably 20% by mass or less, in view of the operability during the treatment. When the glyoxylic acid compound is a glyoxylic acid hydrate, a glyoxylate, or a glyoxyl amide, the content can fall within the above ranges in terms of glyoxylic acid.

The first composition, which is an acidic composition, has a pH of preferably 4.0 or less, more preferably 3.0 or less, still more preferably 2.5 or less, in view of the sufficient hair-straightening effect, and also preferably 0.5 or more, more preferably 1.0 or more, in view of safety. The pH of the first composition can be adjusted by using a known alkali agent, preferably sodium hydroxide or a known acid other than glyoxylic acid compounds. The pH is a value obtained by measuring the composition at 25°C condition using a glass electrode hydrogen ion concentration meter F-52 (manufactured by Horiba, Ltd.), the electrode of which is placed directly into the composition during the measurement.

As mentioned above, conventional methods for permanently changing a hair shape or straightening hair, which are based on breakage and restructuring of disulfide crosslinkages, comprise cleavage of disulfide bonds by, for example, a sulfur reducing agent, an alkali agent, followed by change of the hair shape and formation of new bonds. These new bonds are disulfide bonds or thioether bonds formed by the action of an oxidizing agent after cleavage of the disulfide bonds. Unlike such permanent hair straightening methods, the present invention utilizes neither cleavage of the disulfide bonds nor fixing of bonds in a new shape. Accordingly, the first composition employed in the present invention does not require the presence of a sulfur reducing agent. However, the sulfur reducing agent in an amount of up to 2% by mass based on the total amount of the composition would not interfere with the hair straightening performance of the composition. Accordingly, the content of the sulfur reducing agent in the first composition is preferably less than 2% by mass, more preferably less than 1% by mass, and the first composition preferably comprises no sulfur reducing agent.

The first composition preferably comprises water as a medium. Additionally, the first composition can suitably comprise further ingredients such as surfactants and/or conditioning components defined below, and can suitably take the form of solution, emulsion, cream, paste, gel, or mousse.

### Second Composition (Deodorizing Composition)

The second composition is used for eliminating a distinct odor generated by performing the hair straightening treatment employing the first composition. The pH of the second composition is 9.0 or more, preferably 9.5 or more, more preferably 9.75 or more, still more preferably 10.0 or more, even more preferably 10.5 or more, and also preferably 13.0 or less, more preferably 12.5 or less, still more preferably 12.0 or less, even more preferably 11.5 or less, in view of deodorization of the hair and prevention of damage to the hair. The pH is a value obtained by measuring the composition at 25°C using a glass electrode hydrogen ion concentration meter F-52 (manufactured by Horiba, Ltd.), the electrode of which is placed directly into the composition during the measurement.

Examples of the pH adjusting agent used for adjusting the pH of the second composition to the above pH include alkali metal hydroxides such as sodium hydroxide and potassium hydroxide; ammonia and its salts; alkanolamines such as monoethanolamine, diethanolamine, triethanolamine, isopropanolamine, 2-aminobutanol, and 2-amino-2-methylpropan-1,3-diol. Of these, alkali metal hydroxides are preferred from the viewpoint of preventing odors resulting from these pH adjusting agents themselves. Also, the second composition is preferably a buffer solution in order to sufficiently exhibit the effect of the present invention even when areas to which only a small amount of the second composition is applied are formed due to uneven application of the second composition to the hair, or when a portion of the first composition is left unwashed. In such cases, a phosphate such as disodium hydrogen phosphate and sodium dihydrogen phosphate; a carbonate such as potassium carbonate, sodium carbonate, potassium hydrogen carbonate, and sodium hydrogen carbonate; a borate such as sodium borate and potassium borate; or other publicly known acid is preferably contained. In view of the sufficient deodorizing effect, a phosphate or a carbonate is more preferably contained.

Since the hair treatment of the present invention is intended to exhibit the effect of the invention of the instant application with scarcely changing the color of the hair, the second composition comprises substantially no oxidizing agent such as hydrogen peroxide and persulfates. The wording "Comprises substantially no oxidizing agent" means that the content of the oxidizing agent is less than 0.5% by mass, preferably less than 0.1% by mass, still more preferably less than 0.05% by mass, still more preferably less than 0.01% by mass, still more preferably less than 0.005% by mass, still more preferably less than 0.001% by mass based on the total amount of the second composition.

Since the hair treatment of the present invention is intended to exhibit the effect of the invention of the instant application with scarcely changing the color of the hair, the second composition preferably comprises substantially no dye. The dye herein means direct dyes such as acid dyes, anionic dyes, nitro dyes, disperse dyes, and cationic dyes as well as oxidative dye intermediates (precursors and couplers) and mixtures thereof. Also, the wording "comprises substantially no dye" means that the content of the dye is preferably less than 0.5% by mass, more preferably less than 0.1% by mass, still more preferably less than 0.05% by mass, still more preferably less than 0.01% by mass, still more preferably less than 0.005% by mass, still more preferably less than 0.001% by mass based on the total amount of the second composition.

The second composition preferably comprises water as a medium. Additionally, the second composition can suitably comprise further ingredients such as surfactants and/or conditioning components defined below. Additionally, suitable examples of the possible form of the formulation include solution, emulsion, cream, paste, gel, shampoo, conditioner, treatment, rinse, leave-in treatment, mist, spray, and foam.

The viscosity value of the second composition is preferably 10 mPa·s or more, more preferably 100 mPa·s or more, still more preferably 200 mPa·s or more, still more preferably 1,000 mPa·s or more, in view of preventing the composition from dripping during application, and also preferably 300,000 mPa·s or less, more preferably 200,000 mPa·s or less, still more preferably 150,000 mPa·s or less, still more preferably 100,000 mPa·s or less, from the point of applicability.

The viscosity of the second composition herein refers to a value measured with a B-type viscometer at 30°C. Specifically, when the viscosity of the composition is 20,000 mPa·s or less, a B-type viscometer TVB-10M (manufactured by Toki Sangyo Co., Ltd.) is used, and when the viscosity of the second composition is more than 20,000 mPa·s, a B-type viscometer TVB-10R (manufactured by Toki Sangyo Co., Ltd.) is used in combination with T-BAR STAGE TS-10 (manufactured by Toki Sangyo Co., Ltd.). The viscosity is to be measured using the following rotors and the number of rotations after rotation for 60 seconds at 30°C. The number of rotations to be used is 30 rpm for the viscosity of the composition of 10 mPa·s or less, 60 rpm for the viscosity of more than 10 mPa·s and 20 mPa·s or less, 30 rpm for the viscosity of more than 20 mPa·s and 20,000 mPa·s or less, 5 rpm for the viscosity of more than 20,000 mPa·s. Also, the rotor to be used is L/Adp for the viscosity of the composition of 10 mPa·s or less, M1 for the viscosity of more than 10 mPa·s and 200 mPa·s or less, M2 for the viscosity of more than 200 mPa·s and 1,000 mPa·s or less, M3 for the viscosity of more than 1,000 mPa·s and 4,000 mPa·s or less, M4 for the viscosity of more than 4,000 mPa·s and 20,000 mPa·s or less, T-B for the viscosity of more than 20,000 mPa·s and 160,000 mPa·s or less, and T-C for the viscosity of more than 160,000 mPa·s.

### Thickening Polymer

The first composition and the second composition can comprise a thickening polymer which can be used in hair cosmetics as long as it does not impair the effects of the present invention. Examples of the thickening polymer include cationic thickening polymers, nonionic thickening polymers, anionic thickening polymers.

Examples of the cationic thickening polymer include natural or semisynthetic cationic polysaccharides, and synthetic polymers which comprise an amino group or an ammonium group in the side chain of the polymer chain or which comprises a diallyl quaternary ammonium salt as a constituent unit.

Specific examples of the cationic polysaccharides include cationized cellulose derivatives, cationized guar gum derivatives, hydroxypropyl chitosan, and chitosan dl-pyrrolidone carboxylates.

Examples of the synthetic cationic polymer which comprises an amino group or an ammonium group in the side chain of the polymer chain include synthetic cationic polymers comprising, for example, trialkyl aminoalkyl(meth)acrylate, trialkyl aminoalkyl(meth)acrylamide, (meth)acrylamide, vinylamine as a constituent unit. Specific examples include a polymer of methacryloyloxy ethylene trimonium chloride (INCI name: Polyquaternium-37), a (acrylic acid/methyl acrylate/3-methacryloylamino propyl trimethylammonium chloride) copolymer (INCI name: Polyquaternium-47), a (acrylic acid/acrylamide/methyl methacrylamido propyl trimethylammonium chloride) copolymer (INCI name: Polyquaternium-53), a (dimethylacrylamide/ethyltrimonium chloride methacrylate) copolymer, and a (vinylamine/vinyl alcohol) copolymer.

Examples of the nonionic thickening polymer include synthetic nonionic polymers comprising natural or semisynthetic nonionic polysaccharides, vinyl alcohol, or oxyalkylene as a constituent unit.

Specific examples of the natural or semisynthetic nonionic polysaccharides include water-soluble natural polysaccharides such as starch, guar gum, locust bean gum, and glucomannan, and water-soluble hydroxyalkylated polysaccharides obtained by allowing alkylene oxide to react with cellulose, starch, guar gum, or locust bean gum. Specific examples include guar gum, pullulan, hydroxyethyl cellulose, methyl hydroxyethyl cellulose, hydroxypropyl cellulose, and hydroxypropyl methylcellulose.

Specific examples of the synthetic nonionic thickening polymer comprising vinyl alcohol and polyoxyalkylene as constituent units include polyvinyl alcohol and highly polymerized polyethylene glycol, and a (PEG-240/decyltetradeceth-20/HDI) copolymer.

Examples of the anionic thickening polymer include polyacrylic acid, acrylic acid-methacrylic acid copolymer, hydrolysates of a lower alkyl vinyl ether/maleic anhydride copolymer partially crosslinked with a terminal-unsaturated diene compound or monoalkyl esters of the hydrolysate, carrageenan, xanthan gum, dehydro xanthan gum, welan gum, hydroxypropyl xanthan gum, stearoxy sodium PG hydroxyethylcellulose sulfonate, and (hydroxyethyl acrylate/acryloyl dimethyl taurine Na) copolymer.

Examples of the synthetic cationic polymer which comprises a diallyl quaternary ammonium salt as a constituent unit include a polymer of diallyldimethylammonium chloride (INCI name: Polyquaternium-6), a (dimethyldiallylammonium chloride/acrylamide) copolymer (INCI name: Polyquaternium-7), a (acrylic acid/diallyldimethylammonium chloride) copolymer (INCI name: Polyquaternium-22), and an (acrylamide/acrylic acid/diallyldimethylammonium chloride) copolymer (INCI name: Polyquaternium-39).

Thickening polymers may be used alone or in combinations of two or more. When the first composition and/or the second composition comprise a thickening polymer, the content is, based on the total amount of each of the first composition and the second composition, preferably 0.01% by mass or more, more preferably 0.05% by mass or more, still more preferably 0.1% by mass or more, in view of imparting satisfactory-spread and affinity to the hair on application and also preferably 3% by mass or less, more preferably 2.5% by mass or less, still more preferably 2% by mass or less, in view of imparting satisfactory operability during the treatment.

### Surfactant

The first composition and the second composition can comprise a surfactant which can be used in hair cosmetics as long as it does not impair the effects of the present invention. As the surfactant, any of cationic surfactants, nonionic surfactants, amphoteric surfactants, and anionic surfactants can be used. Two or more of surfactants can be used in combinations.

The cationic surfactant is preferably a mono-long-chain-alkyl quaternary ammonium salt having one C₈ to C₂₄ alkyl group and three C₁ to C₄ alkyl groups.

Preferably, at least one mono-long-chain-alkyl quaternary ammonium surfactant is selected from the compounds represented by the following formula: 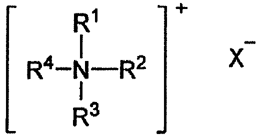
wherein R¹ represents a saturated or unsaturated branched or linear alkyl chain having 8 to 22 carbon atoms, R⁵-CO-NH-(CH₂)ₙ-, or R⁵-CO-O-(CH₂)ₙ- (R⁵ is a saturated or unsaturated branched or linear alkyl chain having 7 to 21 carbon atoms, and n in an integer of 1 to 4),
R², R³, and R⁴ each independently represent an alkyl group having 1 to 4 carbon atoms, a hydroxylalkyl chain having 1 to 4 carbon atoms, or an ethoxy or propoxy group (the number of ethoxy or propoxy groups ranges from 1 to 4), and
X represents a chloride, bromide, methosulfate or ethosulfate.

Specific examples of the cationic surfactant include long-chain quaternary ammonium compounds such as cetyltrimethylammonium chloride, myristyltrimethylammonium chloride, behentrimonium chloride, trimethylcetylammonium bromide, stearyltrimethylammonium chloride, stearyltrimonium chloride, and stearamidopropyltrimonium chloride. These can be used alone or in combinations of two or more.

Examples of the nonionic surfactant include polyoxy-C₁₋₄ alkylene C₈₋₂₄ alkylethers, polyoxy-C₁₋₄ alkylene C₈₋₂₄ alkenylethers, higher (C₁₂₋C₂₄) fatty acid sucrose esters, polyglycerin C₈₋₂₄ fatty acid esters, higher (C₁₂₋C₂₄) fatty acid mono- or diethanolamides, polyoxyethylene hydrogenated castor oil, polyoxyethylene sorbitan C₈₋₂₄ fatty acid esters, polyoxyethylene sorbit C₈₋₂₄ fatty acid esters, C₈₋₂₄ alkylsaccharide surfactants, C₈₋₂₄ alkylamine oxides, and C₈₋₂₄ alkylamidoamine oxides.

Examples of the amphoteric surfactant include imidazoline surfactants, carbobetaine surfactants, amidobetaine surfactants, sulfobetaine surfactants, hydroxysulfobetaine surfactant, and amidosulfobetaine surfactants.

Examples of the anionic surfactant include alkylbenzenesulfonates, alkyl or alkenyl ether sulfates, alkyl or alkenyl sulfates, olefin sulfonates, alkanesulfonates, saturated or unsaturated fatty acid salts, alkyl or alkenyl ether carboxylates, α-sulfo fatty acid salts, N-acylamino acid type surfactants, phosphoric acid mono- or diester type surfactants, and sulfosuccinates. Examples of the alkyl ether sulfate include polyoxyethylene alkyl ether sulfates.

The surfactants can be used alone or in combinations of two or more. When the first composition and/or the second composition comprise a surfactant, the content is, based on the total amount of each of the first composition and the second composition, preferably 0.05% by mass or more, more preferably 0.1% by mass or more, and also preferably 10% by mass or less, more preferably 5% by mass or less.

### Conditioning Component

The first composition and the second composition can comprise a conditioning component which can be used in hair cosmetics as long as it does not impair the effects of the present invention. The conditioning component is an oil or a polymer which adheres to the hair and improves the feel and the manageability of the hair.

When the first composition and/or the second composition comprise a conditioning component, the total content is, based on the total amount of each of the first composition and the second composition, preferably 0.01% by mass or more, more preferably 0.05% by mass or more, still more preferably 0.1% by mass or more, and also preferably 30% by mass or less, more preferably 20% by mass or less, still more preferably 10% by mass or less.

Examples of the conditioning component generally include silicones, organic conditioning oils (for example, hydrocarbon oils, polyolefins, fatty acid esters, and natural oils), and higher alcohols. The first composition and the second composition can comprise a single conditioning component or a combination of two or more of the components.

### Silicone

The first composition and the second composition preferably comprise a silicone to improve the feel of use. Examples of the silicone include dimethylpolysiloxane and modified silicones (for example, amino-modified silicones, fluorine-modified silicones, alcohol-modified silicones, polyether-modified silicones, epoxy-modified silicones, or alkyl-modified silicones), and dimethylpolysiloxane, polyether-modified silicones, and amino-modified silicones are preferred.

The dimethylpolysiloxane may be any cyclic or non-cyclic dimethylsiloxane polymer.

The polyether-modified silicone may be any silicone having a polyoxyalkylene group, and the group constituting the polyoxyalkylene group may be an oxyethylene group or an oxypropylene group.

The amino-modified silicone may be any silicone having an amino group or an ammonium group, and examples thereof include amino-modified silicone oils having all or a part of the terminal hydroxyl groups capped with, for example, a methyl group, and amodimethicone which does not have the terminals capped. A preferred example of the amino-modified silicone may be a compound represented by the following formula: 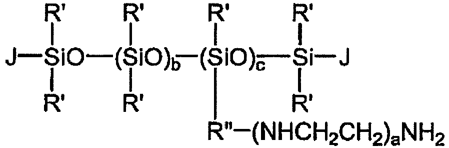 wherein R' represents a hydrogen atom, a hydroxyl group, or R^{z}, R^{z} represents a substituted or unsubstituted monovalent hydrocarbon group having 1 to 20 carbon atoms, J represents R^{z}, R"-(NHCH₂CH₂)ₐNH₂, OR^{z}, or a hydroxyl group, R" represents a divalent hydrocarbon group having 1 to 8 carbon atoms, a represents a number from 0 to 3, and b and c represent numbers, the sum of b and c satisfying a number average of 10 or more and less than 20,000, preferably 20 or more and less than 3000, more preferably 30 or more and less than 1000, and still more preferably 40 or more and less than 800.

When the first composition and/or the second composition comprise a silicone, the content is, based on the total amount of each of the first composition and the second composition, preferably 0.1% by mass or more, more preferably 0.2% by mass or more, still more preferably 0.5% by mass or more, and also preferably 20% by mass or less, more preferably 10% by mass or less, still more preferably 5% by mass or less.

### Organic Conditioning Oil

The first composition and the second composition can contain organic conditioning oil to improve the feel of use. The organic conditioning oil suitably used as a conditioning component is preferably a low-viscosity and water-insoluble liquid, and is selected from the group consisting of hydrocarbon oils having at least 10 carbon atoms, polyolefins, fatty acid esters, natural oils, and mixtures thereof. The viscosity of these organic conditioning oils is preferably 1 mPa·s or more, more preferably 2 mPa·s or more, and also preferably 200 mPa·s or less, more preferably 100 mPa·s or less, still more preferably 50 mPa·s or less. For the determination of the viscosity, a capillary viscometer may be used. The viscosity of the organic conditioning oil herein refers to a value measured at 40°C using a capillary viscometer.

Examples of the hydrocarbon oil include cyclic hydrocarbons, linear aliphatic hydrocarbons (saturated or unsaturated), and branched aliphatic hydrocarbons (saturated or unsaturated), and polymers or mixtures thereof are also included. The linear hydrocarbon oil preferably has 12 to 19 carbon atoms. Examples of the branched hydrocarbon oil include hydrocarbon polymers preferably having more than 19 carbon atoms.

Examples of polyolefin include liquid polyolefins, more preferably liquid poly-α-olefins, still more preferably hydrogenated liquid poly-α-olefins. The polyolefin used in the present invention is prepared by polymerizing an olefin monomer having preferably 4 or more carbon atoms, more preferably 6 or more carbon atoms, and also more preferably 14 or less carbon atoms, more preferably 12 or less carbon atoms.

As the fatty acid ester, a fatty acid ester having 10 or more carbon atoms can be used, for example. Examples of such a fatty acid ester include esters having a hydrocarbon chain derived from a fatty acid and an alcohol (for example, monoesters, polyhydric alcohol esters, or di- and tricarboxylic acid esters). The hydrocarbon group of these fatty acid esters may have other compatible functional group such as an amide group or an alkoxy group as a substituent, or the hydrocarbon group may be covalently bonded to those functional groups. More specifically, an alkyl and alkenyl ester of a fatty acid having 10 to 22 carbon atoms, a carboxylic acid ester of an alkyl and/or alkenyl alcohol having 10 to 22 carbon atoms, and a mixture thereof are suitably used. Specific examples of these fatty acid esters include isopropyl isostearate, hexyl laurate, isohexyl laurate, isohexyl palmitate, isopropyl palmitate, decyl oleate, isodecyl oleate, hexadecyl stearate, decyl stearate, dihexadecyl adipate, lauryl lactate, myristyl lactate, cetyl lactate, oleyl stearate, oleyl oleate, oleyl myristate, lauryl acetate, cetyl propionate and dioleyl adipate.

Further suitable oil components are natural oils such as, for example, liquid paraffin, natural triglycerides. Suitable natural triglycerides are argan oil, shea butter oil, karite oil, olive oil, almond oil, avocado oil, ricinus oil, coconut oil, palm oil, sesame oil, peanut oil, sunflower oil, peach kernel oil, wheat germ oil, macadamia nut oil, macadamia oil, night primrose oil, jojoba oil, castor oil, soya oil, lanolin, passiflora oil, black cumin oil, borage oil, grapeseed oil, hempseed oil, kukui nut oil, and rosehip oil.

The organic conditioning oils can be used alone or in combinations of two or more. The total content is, based on the total amount of each of the first composition and the second composition, preferably 0.1% by mass or more, more preferably 0.2% by mass or more, still more preferably 0.5% by mass or more, and also preferably 20% by mass or less, more preferably 10% by mass or less, still more preferably 5% by mass or less.

### Higher alcohol

The first composition and the second composition can comprise a higher alcohol having 8 or more carbon atoms in view of improving the sense of touch and stability. The number of carbon atoms of the higher alcohol is preferably 8 or more, more preferably 16 or more, and also preferably 22 or less. Specific examples thereof include cetyl alcohol, stearyl alcohol, behenyl alcohol, and mixtures thereof.

The higher alcohols can be used alone or in combinations of two or more. The content is, based on the total amount of each of the first composition and the second composition, preferably 0.1% by mass or more, more preferably 0.2% by mass or more, still more preferably 0.5% by mass or more, and also preferably 20% by mass or less, more preferably 10% by mass or less, still more preferably 5% by mass or less.

Additionally, polyols may suitably be comprised in the compositions. Examples of the polyalkylene glycol include polyethylene glycol and polypropylene glycol, and mixtures of the two may be used. Copolymers of ethylene oxide and propylene oxide may also be used.

Furthermore, the first composition and the second composition can comprise further ingredients conventionally used in the field of cosmetics, such as preservatives, chelating agents, stabilizers, antioxidants, plant extracts, ultraviolet absorbers, vitamins, dyes, and fragrances.

A hair treatment kit comprising the first composition and the second composition described above also can be provided.

### Hair Treatment Method

The hair treatment method of the present invention involves hair straightening steps (steps 1 to 3) and deodorizing steps (steps 4 to 5).

The straightening steps achieve semipermanent hair straightening by utilizing the glyoxylic acid compound as the active component. This straightening effect does not depend on cleaving the disulfide bonds by reduction or the action of strong alkali. Accordingly, use of a reducing composition or an alkaline relaxer (lanthionization agent) is not required.

In the step 1, the first composition (hair straightening composition) is applied to the hair. The mass ratio between the hair and the first composition (hair:first composition) is preferably 0.5:2 to 2:0.5, more preferably 0.5:1 to 1:0.5, still more preferably about 1:1, in view of the sufficient hair straightening effect.

In the step 2, the hair having the first composition applied thereto is left to stand preferably at a temperature of 20 to 45°C, more preferably at room temperature (25°C). The leaving time is preferably one minute or more, more preferably five minutes or more, still more preferably 10 minutes or more, still more preferably 15 minutes or more, and also preferably 120 minutes or less, more preferably 90 minutes or less, still more preferably 60 minutes or less, still more preferably 45 minutes or less, in view of the sufficient hair straightening effect. The time for leaving the first composition to stand on the hair depends on the type of the hair, that is, whether the hair is damaged or resistant. The leaving time refers to the time from the completion of the step 1 to the start of the next step.

After the step 2 and before the step 3, the hair may be optionally heat-dried in order to avoid an excessive steam generation while the hair is heated in the step 3. Typically, a heating device which does not allow the hair surface temperature to exceed 120°C on heating, such as a hair dryer is preferably used for this purpose. It is preferable to dry the hair under continuous combing with, for example, a hand, a comb, in order to prevent entanglement of the hair.

Furthermore, the hair may optionally be rinsed with water and dried once again between the step 2 and the step 3.

In the step 3, the hair is heated with a hair iron, preferably a straight iron can be used for this purpose. In order to prevent damage to the hair, the surface temperature of the plates of the hair iron is 250°C or less, preferably 230°C or less. For achieving a sufficient hair straightening, the temperature of the hair iron is 130°C or more, preferably 150°C or more, more preferably 170°C or more. The surface temperature of the hair iron can be preferably 180 ± 50°C, more preferably 170 to 230°C.

After the step 3, the hair may be optionally rinsed with water. After this rinsing, the hair may be optionally shampooed. After this rinsing or shampooing, the hair may be optionally dried.

The deodorizing step is performed after the hair straightening step.

In the step 4, the second composition (deodorizing composition) is applied to the hair. The mass ratio between the hair and the second composition (hair:second composition) is preferably 0.5:2 to 2:0.5, more preferably 0.5:1 to 1:0.5, still more preferably about 1:1.

In the step 5, the hair having the second composition applied thereto is left to stand for a predetermined time. The leaving time here is preferably one minute or more, more preferably five minutes or more, and also preferably 45 minutes or less, more preferably 30 minutes or less, still more preferably 20 minutes or less, in view of convenience for performing the treatment and prevention of reduction in the hair strength.

After the step 5, the hair may be left to stand or dried as it is without rinsing the hair, or the hair may be optionally rinsed with water. After this rinsing, the hair may be optionally shampooed. After this rinsing or shampooing, the hair may be optionally dried. In view of convenience of the treatment and reduction in the treatment time, it is preferred to leave the hair to stand or dry the hair as it is without rinsing the hair. In view of a higher deodorizing effect after the treatment and more natural feel of the hair after the treatment, it is preferred to rinse the hair with water and optionally shampoo and dry the hair.

In connection with the aforementioned embodiments, preferred aspects of the present invention will be further disclosed hereinbelow.
<1> A hair treatment method comprising steps 1 to 5 to be performed in the following order:
   1. a step of applying a first composition to hair, wherein the first composition is acidic and comprises one or more member selected from the group consisting of glyoxylic acid, glyoxylic acid hydrates, glyoxylates, and glyoxyl amides;
   2. a step of leaving the hair having the first composition applied thereto to stand;
   3. a step of heating the hair with a hair iron having a temperature of 130 to 250°C;
   4. a step of applying a second composition to the hair, wherein the second composition comprises substantially no oxidizing agent, i.e., the content of oxidizing agent being less than 0.5% by mass, and has a pH of 9.0 or more; and
   5. a step of leaving the hair having the second composition applied thereto to stand.
<2> The hair treatment method according to <1>, wherein the second composition is a buffer solution.
<3> The hair treatment method according to <1> or <2>, wherein the pH of the second composition at 25°C is 9.0 or more, preferably 9.5 or more, preferably 9.75 or more, more preferably 10.0 or more, still more preferably 10.5 or more, and also preferably 13.0 or less, more preferably 12.5 or less, still more preferably 12.0 or less, still more preferably 11.5 or less.
<4> The hair treatment method according to any of <1> to <3>, wherein the content of the oxidizing agent in the second composition is less than 0.1% by mass, preferably less than 0.05% by mass, more preferably less than 0.01% by mass, still more preferably less than 0.005% by mass, still more preferably less than 0.001% by mass.
<5> The hair treatment method according to any of <1> to <4>, wherein the content of the dye in the second composition is preferably less than 0.5% by mass, more preferably less than 0.1% by mass, still more preferably less than 0.05% by mass, still more preferably less than 0.01% by mass.
<6> The hair treatment method according to any of <1> to <5>, wherein in the step 5, the time for leaving the hair having the second composition applied thereto to stand is preferably one minute or more, more preferably five minutes or more, and also preferably 45 minutes or less, more preferably 30 minutes or less, still more preferably 20 minutes or less.
<7> The hair treatment method according to any of <1> to <6>, wherein after the step 5, the hair is preferably left to stand or dried without rinsing the hair with water.
<8> The hair treatment method according to any of <1> to <6>, wherein after the step 5, the hair is preferably rinsed with water.
<9> The hair treatment method according to any of <1> to <8>, wherein after the step 2 and before the step 3, the hair is preferably heat-dried.
<10> The hair treatment method according to any of <1> to <9>, wherein the high temperature heating device used in the step 3 is a hair iron having a plate surface temperature of 230°C or less and 150°C or more, more preferably 170°C or more.
<11> The hair treatment method according to any of <1> to <10>, wherein after the step 3, the hair is preferably rinsed with water.
<12> The hair treatment method according to any of <1> to <11>, wherein the content of the compound selected from the group consisting of glyoxylic acid, glyoxylic acid hydrates, glyoxylates, and glyoxyl amides in the first composition is preferably 0.1% by mass or more, more preferably 0.5% by mass or more, still more preferably 1% by mass or more, still more preferably 2.5% by mass or more, and also preferably 40% by mass or less, more preferably 30% by mass or less, still more preferably 25% by mass or less, still more preferably 20% by mass or less in terms of glyoxylic acid.
<13> The hair treatment method according to any of <1> to <12>, wherein the pH of the first composition at 25°C is preferably 4.0 or less, more preferably 3.0 or less, still more preferably 2.5 or less, and also preferably 0.5 or more, more preferably 1.0 or more.
<14> Use of a hair treatment kit comprising a first composition which is acidic and comprises one or more member selected from the group consisting of glyoxylic acid, glyoxylic acid hydrates, glyoxylates, and glyoxyl amides, and a second composition which comprises substantially no oxidizing agent, i.e., the content of oxidizing agent being less than 0.5% by mass, and has a pH of 9.0 or more, for straightening hair using the procedure as defined in any one of <1> to <13>

### Examples

### Examples 1 to 8 and Comparative Examples 1 to 3

Treatment agents A to I shown in the following Table 1 were prepared, and various evaluations were performed. Additionally, the pH of the composition was measured using a glass electrode hydrogen ion concentration meter F-52 (manufactured by Horiba, Ltd.). During the measurement, the electrode of meter was placed directly into the sample, of which temperature had been adjusted to 25°C.

**[Table 1]**

| (% by mass) | Treatment agent | | | | | | | | |
|---|---|---|---|---|---|---|---|---|---|
| | First composition (hair straightening composition) | Second composition (deodorizing composition) | | | | | | | |
| | A | β*¹ | C | D | E | F | G | H | I |
| Glyoxylic acid | 10 | - | - | - | - | - | - | - | - |
| Sodium hydroxide | *2 | *2 | *2 | *2 | *2 | *2 | *2 | *2 | *2 |
| Sodium hydrogen carbonate | - | - | - | 0.21 | 0.21 | - | - | - | - |
| Sodium hydrogen phosphate | - | - | - | - | - | 0.355 | 0.355 | - | - |
| Boric acid | - | - | - | - | - | - | - | 0.309 | 0.309 |
| Potassium chloride | - | - | - | - | - | - | - | 0.373 | 0.373 |
| Water | Balance | Balance | Balance | Balance | Balance | Balance | Balance | Balance | Balance |
| Total | 100 | 100 | 100 | 100 | 100 | 100 | 100 | 100 | 100 |
| pH | 2.0 | 8.0 | 10.0 | 10.0 | 11.0 | 11.0 | 12.0 | 9.0 | 11.0 |

| | | | | | | | | | |
|---|---|---|---|---|---|---|---|---|---|
| *1: Although composition B does not belong to a second composition for the present invention, is listed for convenience. *2: Amount for adjusting the pH | | | | | | | | | |

### Tresses for Evaluation

Caucasian hair having no chemical treatment history was used to make tresses having a length of 8 cm, a width of 2 cm, and weight of 1.5 g, which were used for evaluation.

### [Example 1]

### Hair Straightening Treatment

The treatment agent A was applied to the tress for evaluation in a mass ratio of 1:1 and rubbed onto the tress, which was entirely covered and sealed with wrap and was left to stand at room temperature for 20 minutes. Subsequently, the wrap was removed, and the tress was dried with hot air from a hair dryer (manufactured by Panasonic Corporation, model No. EH5101P) for one minute. Subsequently, the tress was heated with a straight iron heated to actual measured temperature of 230°C (manufactured by Taiff Corporation, model No. TITANIUM450). Immediately thereafter, the hair was rinsed with water. After 1.5 g of a model shampoo of which formulation is shown below was applied to the hair, the hair was washed for one minute, rinsed with tap water at 40°C for 30 seconds, and then towel-dried for extra moisture. The temperature of the straight iron was measured by using TX10 manufactured by Yokogawa Meters & Instruments Corporation.

### Treatment with Deodorizing Composition

The treatment agent C shown in Table 1 was applied to the hair after the above hair straightening treatment such that the mass ratio between the hair and the composition (hair:second composition) was 1:1. The entire tress was covered and sealed with wrap and was left to stand at room temperature for about five minutes. Subsequently, the hair was rinsed with water at 40°C and towel-dried. This tress was subjected to evaluation for the deodorizing effect immediately after and the deodorizing effect after one week in accordance with the evaluation method shown below. Additionally, the breaking strength of the hair immediately after the treatment was measured.

### Model Shampoo Formulation

| Ingredient (% by mass) | |
|---|---|
| Sodium polyoxyethylene(2.5) lauryl ether sulfate | 15.5 |
| Lauroyl diethanolamide | 2.28 |
| Disodium edetate | 0.1 |
| Sodium benzoate | 0.5 |
| Oxybenzone | 0.03 |
| Phosphoric acid | 0.075 |
| Dibutylhydroxytoluene | 0.01 |
| Sodium chloride | 0.8 |
| Purified water | Balance |
| Total | 100 |

### Method for Evaluating Deodorizing Effect Immediately after Treatment

The odor of the tress immediately after the treatment was smelled and evaluated based on the following five grades. The evaluation was performed by five panelists, and the average score of the evaluation is shown in Table 2.
1: Smells very strongly (no deodorizing effect)
2: Smells slightly strongly
3: Slightly smells
4: Scarcely smells
5: Not smells at all

### Method for Evaluating Deodorizing Effect after One Week

The tress subjected to the deodorizing effect evaluation immediately after the treatment was dried with a hair dryer (manufactured by Panasonic Corporation, model No. EH5101P) for one minute and allowed to stand still at room temperature for one week. Subsequently, this tress was moistened with tap water at 40°C for one minute, towel-dried, and then, subjected to evaluation in accordance with the same method and evaluation criteria as those of the deodorizing effect immediately after. The evaluation was performed by five panelists, and the average score of the evaluation is shown in Table 2.

### Method for Measuring Breaking strength

For Examples 1, 3, and 4, the breaking strength of the hair immediately after the treatment was measured. Twenty hairs were randomly selected from the tress immediately after the treatment, and the breaking strength of each of the hairs was measured with a tensile tester (manufactured by Dia-Stron Limited, Miniature Tensile Tester 675/680), followed by calculating the average value thereof. First, both ends were fixed such that the interval of the hair was 3 cm, and the sectional area of the hair was measured. Then, the hair was immersed in ion-exchanged water for 30 minutes or more. Thereafter, the hair was elongated at a rate of 18 mm/minute to be broken. The load per unit sectional area of the hair at break was taken as the breaking strength (expression-1). Breaking strength (Mpa) = Load at break (kg-m/s²)/Hair sectional area (mm²) (Expression-1)

### [Example 2]

Procedures and evaluation in the same manner as in Example 1 were performed except that the leaving time for the treatment agent C was 20 minutes in Example 1.

### [Example 3]

Procedures and evaluation in the same manner as in Example 1 were performed except that the treatment agent D was used as the deodorizing composition in place of the treatment agent C in Example 1.

### [Example 4]

Procedures and evaluation in the same manner as in Example 1 were performed except that the treatment agent E was used as the deodorizing composition in place of the treatment agent C in Example 1.

### [Example 5]

Procedures and evaluation in the same manner as in Example 1 were performed except that the treatment agent F was used as the deodorizing composition in place of the treatment agent C in Example 1.

### [Example 6]

Procedures and evaluation in the same manner as in Example 1 were performed except that the treatment agent G was used as the deodorizing composition in place of the treatment agent C in Example 1.

### [Example 7]

Procedures and evaluation in the same manner as in Example 1 were performed except that the treatment agent H was used as the deodorizing composition in place of the treatment agent C in Example 1.

### [Example 8]

Procedures and evaluation in the same manner as in Example 1 were performed except that the treatment agent I was used as the deodorizing composition in place of the treatment agent C in Example 1.

### [Comparative Example 1]

After a hair straightening treatment in the same manner as in Example 1 was performed, evaluation similar to that of Example 1 was performed without applying the deodorizing composition.

### [Comparative Example 2]

After Procedures in the same manner as in the treatment with the deodorizing composition in Example 1 was performed by using the treatment agent C, Procedures in the same manner as in the hair straightening treatment in Example 1 was performed. Subsequently, evaluation was performed in the same manner as in Example 1.

### [Comparative Example 3]

Procedures and evaluation in the same manner as in Example 1 were performed except that the treatment agent B was used as the deodorizing composition in place of the treatment agent C in Example 1.

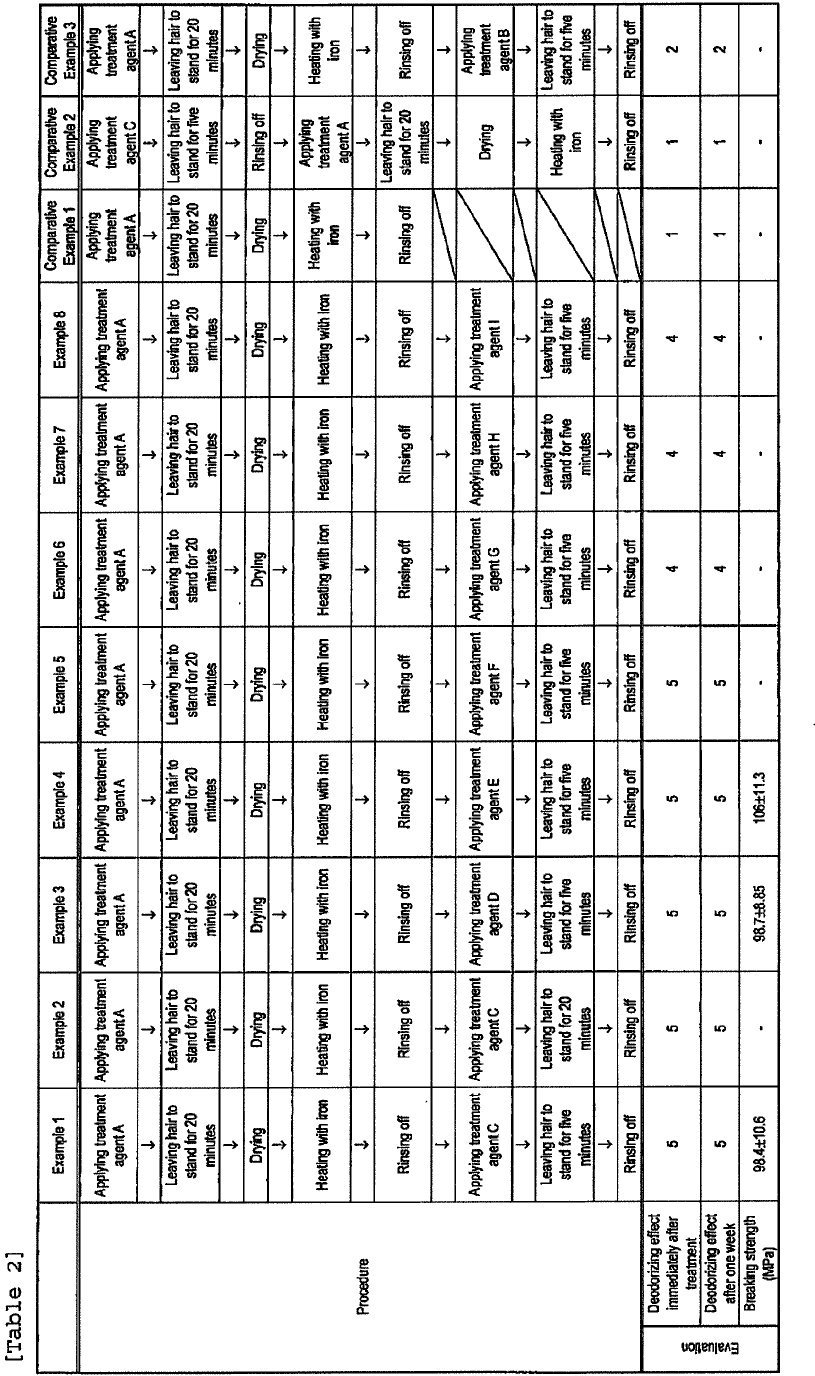

## Claims

1. A hair treatment method comprising steps 1 to 5 to be performed in the following order:
1. a step of applying a first composition to hair, wherein the first composition is acidic and comprises one or more member selected from the group consisting of glyoxylic acid, glyoxylic acid hydrates, glyoxylates, and glyoxyl amides;
2. a step of leaving the hair having the first composition applied thereto to stand;
3. a step of heating the hair with a hair iron having a temperature of 130 to 250°C;
4. a step of applying a second composition to the hair, wherein the second composition comprises substantially no oxidizing agent, i.e., the content of oxidizing agent being less than 0.5% by mass, and has a pH of 9.0 or more; and
5. a step of leaving the hair having the second composition applied thereto to stand.

2. The hair treatment method according to claim 1, wherein the second composition is a buffer solution.

3. The hair treatment method according to claim 1 or 2, wherein in the step 5, the time for leaving the hair having the second composition applied thereto to stand is one minute or more and 45 minutes or less.

4. The hair treatment method according to any of claims 1 to 3, wherein after the step 5, the hair is left to stand or dried without rinsing the hair with water.

5. The hair treatment method according to any of claims 1 to 3, wherein after the step 5, the hair is rinsed with water.

6. The hair treatment method according to any of claims 1 to 5, wherein the dye component contained in the second composition is less than 0.1% by mass.

7. The hair treatment method according to any of claims 1 to 6, wherein the pH of the first composition is 4.0 or less.

8. The hair treatment method according to any of claims 1 to 7, wherein the content of glyoxylic acid in the first composition is 0.1% by mass or more and 40% by mass or less.

9. The hair treatment method according to any of claims 1 to 8, wherein after the step 2 and before the step 3, the hair is heat-dried.

10. The hair treatment method according to any of claims 1 to 9, wherein after the step 3 and before the step 4, the hair is rinsed with water.

11. Use of a hair treatment kit comprising a first composition which is acidic and comprises one or more member selected from the group consisting of glyoxylic acid, glyoxylic acid hydrates, glyoxylates, and glyoxyl amides, and a second composition which comprises substantially no oxidizing agent, i.e., the content of oxidizing agent being less than 0.5% by mass, and has a pH of 9.0 or more, for straightening hair using the procedure as defined in any one of the claims 1 to 10.

## Patentansprüche

1. Haarbehandlungsverfahren, umfassend die Schritte 1 bis 5, die in der nachstehenden Reihenfolge durchzuführen sind:
(1) einen Schritt, in dem eine erste Zusammensetzung auf das Haar aufgebracht wird, wobei die erste Zusammensetzung sauer ist und ein oder mehrere Elemente, ausgewählt aus der Gruppe bestehend aus Glyoxylsäure, Glyoxylsäurehydraten, Glyoxylaten und Glyoxylamiden, umfasst;
(2) einen Schritt, in dem das Haar mit der darauf aufgebrachten ersten Zusammensetzung ruhen gelassen wird;
(3) einen Schritt, in dem das Haar mit einem Haareisen, das eine Temperatur von 130 bis 250°C aufweist, erwärmt wird;
(4) einen Schritt, in dem eine zweite Zusammensetzung auf das Haar aufgebracht wird, wobei die zweite Zusammensetzung im wesentlichen kein Oxidationsmittel umfasst, d.h. der Gehalt an Oxidationsmittel weniger als 0,5 Masse-% beträgt, und einen pH-Wert von 9,0 oder höher aufweist; und
(5) einen Schritt, in dem das Haar mit der darauf aufgebrachten zweiten Zusammensetzung ruhen gelassen wird.

2. Haarbehandlungsverfahren gemäss Anspruch 1, wobei die zweite Zusammensetzung eine Pufferlösung ist.

3. Haarbehandlungsverfahren gemäss Anspruch 1 oder 2, wobei im Schritt 5, die Zeit zum Ruhenlassen des Haares mit der darauf aufgebrachten zweiten Zusammensetzung 1 Minute oder mehr und 45 Minuten oder weniger beträgt.

4. Haarbehandlungsverfahren gemäss einem der Ansprüche 1 bis 3, wobei nach dem Schritt 5, das Haar ruhen gelassen oder getrocknet wird, ohne das Haar mit Wasser zu spülen.

5. Haarbehandlungsverfahren gemäss einem der Ansprüche 1 bis 3, wobei nach dem Schritt 5, das Haar mit Wasser gespült wird.

6. Haarbehandlungsverfahren gemäss einem der Ansprüche 1 bis 5, wobei die Farbstoffkomponente, die in der zweiten Zusammensetzung enthalten ist, weniger als 0,1 Masse-% beträgt.

7. Haarbehandlungsverfahren gemäss einem der Ansprüche 1 bis 6, wobei der pH-Wert der ersten Zusammensetzung 4,0 oder weniger beträgt.

8. Haarbehandlungsverfahren gemäss einem der Ansprüche 1 bis 7, wobei der Gehalt an Glyoxylsäure in der ersten Zusammensetzung 0,1 Masse-% oder mehr und 40 Masse-% oder weniger beträgt.

9. Haarbehandlungsverfahren gemäss einem der Ansprüche 1 bis 8, wobei nach dem Schritt 2 und vor dem Schritt 3 das Haar wärmegetrocknet wird.

10. Haarbehandlungsverfahren gemäss einem der Ansprüche 1 bis 9, wobei nach dem Schritt 3 und vor dem Schritt 4 das Haar mit Wasser gespült wird.

11. Verwendung eines Haarbehandlungskits, umfassend eine erste Zusammensetzung, die sauer ist und ein oder mehr Elemente, ausgewählt aus der Gruppe bestehend aus Glyoxylsäure, Glyoxylsäurehydraten, Glyoxylaten und Glyoxylamiden, umfasst und eine zweite Zusammensetzung, die im wesentlichen kein Oxidationsmittel umfasst, d.h. der Gehalt an Oxidationsmittel weniger als 0,5 Masse-% beträgt, und einen pH-Wert von 9,0 oder mehr aufweist, zum Glätten der Haare unter Verwendung des in irgendeinem der Ansprüche 1 bis 10 definierten Verfahrens.

## Revendications

1. Procédé de traitement capillaire comprenant les étapes 1 à 5 à effectuer dans l'ordre suivant :
1. une étape d'application d'une première composition aux cheveux, dans lequel la première composition est acide et comprend un ou plusieurs éléments choisis dans le groupe constitué de l'acide glyoxylique, des hydrates de l'acide glyoxylique, des glyoxylates et des amides glyoxyliques ;
2. une étape visant à laisser les cheveux reposer après l'application de la première composition à ceux-ci ;
3. une étape de chauffage des cheveux avec un fer à cheveux ayant une température de 130 à 250 °C ;
4. une étape d'application d'une seconde composition aux cheveux, dans lequel la seconde composition ne comprend sensiblement pas d'agent oxydant, c'est-à-dire que la teneur en agent oxydant est inférieure à 0,5 % en masse, et a un pH de 9,0 ou plus ; et
5. une étape visant à laisser les cheveux reposer après la seconde composition qui leur a été appliquée.

2. Procédé de traitement capillaire selon la revendication 1, dans lequel la seconde composition est une solution tampon.

3. Procédé de traitement capillaire selon la revendication 1 ou 2, dans lequel, à l'étape 5, le temps visant à laisser reposer les cheveux après la seconde composition qui leur a été appliquée est d'une minute ou plus et de 45 minutes ou moins.

4. Procédé de traitement capillaire selon l'une quelconque des revendications 1 à 3, dans lequel, après l'étape 5, on laisse reposer les cheveux ou on les sèche sans rincer les cheveux à l'eau.

5. Procédé de traitement capillaire selon l'une quelconque des revendications 1 à 3, dans lequel, après l'étape 5, les cheveux sont rincés à l'eau.

6. Procédé de traitement capillaire selon l'une quelconque des revendications 1 à 5, dans lequel le composant colorant contenu dans la seconde composition est en quantité inférieure à 0,1 % en masse.

7. Procédé de traitement capillaire selon l'une quelconque des revendications 1 à 6, dans lequel le pH de la première composition est de 4,0 ou moins.

8. Procédé de traitement capillaire selon l'une quelconque des revendications 1 à 7, dans lequel la teneur en acide glyoxylique dans la première composition est de 0,1 % en masse ou plus et de 40 % en masse ou moins.

9. Procédé de traitement capillaire selon l'une quelconque des revendications 1 à 8, dans lequel, après l'étape 2 et avant l'étape 3, les cheveux sont séchés à la chaleur.

10. Procédé de traitement capillaire selon l'une quelconque des revendications 1 à 9, dans lequel, après l'étape 3 et avant l'étape 4, les cheveux sont rincés à l'eau.

11. Utilisation d'un kit de traitement capillaire comprenant une première composition qui est acide et comprend un ou plusieurs éléments choisis dans le groupe constitué de l'acide glyoxylique, des hydrates de l'acide glyoxylique, des glyoxylates et des amides glyoxyliques, et une seconde composition qui ne comprend sensiblement pas d'agent oxydant, c'est-à-dire que la teneur en agent oxydant est inférieure à 0,5 % en masse, et a un pH de 9,0 ou plus pour lisser les cheveux en utilisant la procédure telle que définie dans l'une quelconque des revendications 1 à 10.
